(19) Europäisches Patentamt / European Patent Office / Office européen des brevets

(11) **EP 4 591 848 B1**

(12) **EUROPEAN PATENT SPECIFICATION**

(45) Date of publication and mention
of the grant of the patent:
**17.06.2026 Bulletin 2026/25**

(21) Application number: **24213526.7**

(22) Date of filing: **18.11.2024**

(51) International Patent Classification (IPC):
*A61K 8/34* (2006.01)    *A61K 8/64* (2006.01)
*A61K 8/65* (2006.01)    *A61K 8/9728* (2017.01)
*A61Q 19/00* (2006.01)

(52) Cooperative Patent Classification (CPC):
**A61K 8/345; A61K 8/64; A61K 8/65; A61K 8/9728;
A61Q 19/00;** A61K 2800/5922; A61K 2800/85

(54) **CELL AUTOPHAGY COMPOSITION AND PREPARATION METHOD AND APPLICATION THEREOF**

ZELLAUTOPHAGENZUSAMMENSETZUNG SOWIE HERSTELLUNGSVERFAHREN UND ANWENDUNG DAVON

COMPOSITION D'AUTOPHAGIE CELLULAIRE ET SON PROCÉDÉ DE PRÉPARATION ET SON APPLICATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **23.01.2024 CN 202410095040**

(43) Date of publication of application:
**30.07.2025 Bulletin 2025/31**

(73) Proprietor: **Shaanxi Huikang Bio-Tech Co., Ltd.
Xi'an City, Shaanxi Province 710077 (CN)**

(72) Inventors:
• ZHI, Xubo
  710077 Xi'an City (CN)
• WANG, Zhaowei
  710077 Xi'an City (CN)
• SUN, Xueyuan
  710077 Xi'an City (CN)
• LI, Zhe
  710077 Xi'an City (CN)
• ZHANG, Xiaoxia
  710077 Xi'an City (CN)
• GAO, En
  710077 Xi'an City (CN)

(74) Representative: **Habermann, Hruschka & Schnabel
Patentanwälte
Montgelasstraße 2
81679 München (DE)**

(56) References cited:
WO-A1-2015/170064    CN-A- 115 851 466
CN-A- 116 747 184    KR-A- 20230 120 314

• DATABASE GNPD [online] MINTEL; 21 November 2023 (2023-11-21), ANONYMOUS: "Collagen Tender Silky Body Lotion", XP093271456, retrieved from https://www.gnpd.com/sinatra/recordpage/11234028/ Database accession no. 11234028

**Description**

TECHNICAL FIELD

The present disclosure belongs to the technical field of cosmetic compositions, particularly relates to a cell autophagy composition with whitening effect, and also relates to a preparation method and an application of the cell autophagy composition.

BACKGROUND

Autophagy, meaning self-eating, is a process in which eukaryotic cells degrade cytoplasmic proteins and damaged organelles thereof using lysosomes under the regulation of an autophagy related gene (Atg). The autophagy can prevent cell damage and respond to cytotoxic stimulations, and is a self-protection mechanism of cells. In addition, the autophagy is beneficial to growth and development of cells, protects the cells from metabolic stress and oxidative damage, and plays an important role in maintaining intracellular homeostasis and synthesizing, degrading, and recycling cell products.

Modern people are affected by factors such as stress, environment, diet, and cosmetics, skin cells thereof are severely damaged, and the autophagy ability of the skin cells is seriously impaired. Traditional cosmetics focus primarily on hydration and nourishment, but are incapable of repairing or enhancing cell autophagy, or clean up damaged cells and related substances, causing skin cells to be in a harsh physiological environment for a long time and entering a vicious circle.

CN115851466 A discloses the use of Pichia pastoris, especially the fermentate thereof, for skin care compositions which improve moisturisation, strengthen the skin barrier and upregulate the expression of autophagy-related gene LC3B (claims 1-7). Embodiment 9 shows a face cream comprising the P.pastoris ferment lysate.

SUMMARY

The first object of the present disclosure is to provide a cell autophagy composition, by which the problem that existing cosmetics cannot repair or enhance cell autophagy is addressed.

The second object of the present disclosure is to provide a preparation method for the cell autophagy composition.

The third object of the present disclosure is to provide an application of the cell autophagy composition.

The first technical scheme adopted by the present disclosure provides a cell autophagy composition. The cell autophagy composition contains, in percentage by mass: 0.05% to 0.5% of collagen; 0.5% to 3% of a yeast fermentation product extract; 0.01% to 0.5% of acetyl hexapeptide-8; 0.01% to 0.5% of palmitoyl hexapeptide-12; 0.01% to 0.5% of dipeptide-15; 0.1% to 2% of tripeptide-1; 0.01% to 0.5% of dipeptide-1; 5% to 10% of 1,2-hexanediol; and 0.05% to 0.5% of ethylhexylglycerin, with the remainder being water.

The first technical scheme adopted by the present disclosure is also characterized in that:

the collagen is preferably one or a combination of two of type I collagen and type III collagen; and
the yeast fermentation product extract is Pichia pastoris fermentation product extract.

The second technical scheme adopted by the present disclosure provides a preparation method for the cell autophagy composition. The method includes the following specific steps:

step 1 of dissolving collagen and a yeast fermentation product extract in water to obtain a solution, and stirring the solution uniformly;
step 2 of adding acetyl hexapeptide-8, palmitoyl hexapeptide-12, dipeptide-15, tripeptide-1, and dipeptide-1 into the solution obtained in step 1 to obtain a solution, and stirring the solution uniformly; and
step 3 of filtering and sterilizing the solution obtained in step 2 to obtain an autophagy composition.

The third technical scheme adopted by the present disclosure provides an application of the cell autophagy composition in preparation of cosmetics.

The third technical scheme adopted by the present disclosure is also characterized in:

it is optionally that the cell autophagy composition is mixed with a cosmetic matrix to obtain a cosmetic having a cell autophagy function, the cosmetic matrix is any one of an aqueous solution, a cream, a facial mask, and an essence, and an amount of the cell autophagy composition added is 1% to 10% of the cosmetic in percentage by mass.

The present disclosure has the following beneficial effects:

1) According to the cell autophagy composition of the present disclosure, the addition of collagen can exogenously replenish the lost collagen in the skin and stimulate skin cells to synthesize collagen at the same time. The yeast fermentation product extract is rich in various nutrients required for the growth of skin cells, including substances such as amino acids and polysaccharides, and can repair the damaged skin barrier and stimulate the skin cells to secrete substances such as collagen. The dipeptide-15, the tripeptide-1, the dipeptide-1, and the yeast extract work synergistically to repair damaged cells, enhance the cell autophagy function, metabolize and clean up the waste produced by skin cells as soon as possible, prevent oxidative damage, and accelerate the reuse of useful substances to maintain a healthy and steady-state environment for cells, thereby restoring the skin to a young and healthy state and achieving the purpose of cell autophagy.

2) The cell autophagy composition of the present disclosure is simple in formulations, safe, and remarkable in efficacy, and can be directly used for developing various cosmetic products.

## BRIEF DESCRIPTION OF THE DRAWINGS

FIG. 1 is a graph showing cytotoxicity testing results of compositions of Examples 1 to 3 of the present disclosure;
FIG. 2 is a schematic diagram of an effect of the composition of the present disclosure on expression of a cell barrier related gene; and
FIG. 3 is a schematic diagram of an effect of the composition of the present disclosure on expression of cell autophagy related genes.

## DESCRIPTION OF THE EMBODIMENTS

The technical solutions of the present disclosure will be clearly and completely described below in conjunction with the drawings.

The present disclosure provides a cell autophagy composition consisting of collagen, a yeast fermentation product extract, acetyl hexapeptide-8, palmitoyl hexapeptide-12, dipeptide-15, tripeptide-1, dipeptide-1, 1,2-hexanediol, ethylhexylglycerin, and purified water. The collagen and the yeast fermentation product extract can repair damaged skin cells and restore normal physiological functions of the cells. The acetyl hexapeptide-8 and the palmitoyl hexapeptide-12 can effectively reduce a nerve activity of cells. The dipeptide-15, also known as glycylglycine, is a cell stabilizer. The tripeptide-1 and the dipeptide-1 have cell function regulating effects. Assisted by the penetration-promoting effect of 1,2-hexanediol, the various components work synergistically to enhance autophagy and regulate cells. The composition is simple to prepare, safe, and non-irritating, and can be directly used or added into cosmetics as a functional ingredient.

The present disclosure provides a cell autophagy composition, which has a composition of, by mass percent: 0.05% to 0.5% of collagen; 0.5% to 3% of a yeast fermentation product extract; 0.01% to 0.5% of acetyl hexapeptide-8; 0.01% to 0.5% of palmitoyl hexapeptide-12; 0.01% to 0.5% of dipeptide-15; 0.1% to 2% of tripeptide-1; 0.01% to 0.5% of dipeptide-1; 5% to 10% of 1,2-hexanediol; and 0.05% to 0.5% of ethylhexylglycerin, with the remainder being water.

Exemplarily, the collagen is one or both of type I collagen and type III collagen.

Exemplarily, the yeast fermentation product extract is Pichia pastoris fermentation product extract.

The present disclosure also provides a preparation method for the above-mentioned cell autophagy composition. The specific preparation steps are as follows:

step 1 of dissolving the collagen and the yeast fermentation product extract in water and stirring evenly to obtain a solution;
step 2 of adding acetyl hexapeptide-8, palmitoyl hexapeptide-12, dipeptide-15, tripeptide-1, and dipeptide-1 into the solution obtained in step 1 and stirring evenly to obtain a solution; and
step 3 of filtering and sterilizing the solution obtained in step 2 to obtain the cell autophagy composition.

The present disclosure also provides an application of the cell autophagy composition in preparation of cosmetics.

Exemplarily, the cell autophagy composition is mixed with a cosmetic matrix to obtain a cosmetic with cell autophagy function, the cosmetic matrix is any one of an aqueous solution, a cream, a facial mask, and an essence, and an addition amount of the cell autophagy composition is 1% to 10% by mass percent of the cosmetic.

The technical solutions of the present disclosure are further described by the following Examples.

The formulations with different component contents for Examples 1 to 3 of the present disclosure are shown in Table 1.

The cell autophagy composition consists of the following feedstock components by mass percent.

Table 1 Formulation compositions of cell autophagy compositions in Examples 1 to 3 (by mass percent %)

| Components | Example 1 | Example 2 | Example 3 | Comparative Example |
|---|---|---|---|---|
| Collagen | 0.05% | 0.25% | 0.5% | 0 |
| Yeast fermentation product extract | 3% | 1.5% | 0.5% | 0 |
| Acetyl hexapeptide-8 | 0.01% | 0.25% | 0.5% | 0 |
| Palmitoyl hexapeptide-12 | 0.5% | 0.25% | 0.01% | 0 |
| Dipeptide-15 | 0.01% | 0.25% | 0.5% | 0 |
| Tripeptide-1 | 2% | 1% | 0.1% | 0 |
| Dipeptide-1 | 0.5% | 0.25% | 0.01% | 0 |
| 1,2-hexanediol | 5% | 7.5% | 10% | 10% |
| Glycerin | 0.5% | 0.25% | 0.05% | 0.5% |
| Pure water | to 100% | to 100% | to 100% | to 100% |

The above-mentioned components are processed according to the following steps:

1) dissolving the collagen and the yeast fermentation product extract in water and stirring evenly to obtain a solution;
2) adding hyaluronic acid peptide to the solution obtained in step 1 and stirring evenly, then adding 1,2-hexanediol and ethylhexylglycerin, and stirring evenly to obtain a solution; and
3) filtering and sterilizing the above solution obtained in step 2 to obtain the cell autophagy composition.

The efficacy experiments of the present disclosure are as follows.

Example 4 Testing for cytotoxicity of the formulations

The experimental method is as follows. Human skin fibroblasts (HSFs) were seeded into a 96-well culture plate in an amount of $1 \times 10^4$ cells/well. After 24 hours, the obtained products in Examples 1 to 3 each were diluted with a culture solution according to two gradients of 10% and 1% and then separately added into cells, and a control group (cell + culture medium) and a blank control group (culture medium) were set. After culturing for 24 hours, 10 $\mu$L of an MTT solution (5 g•L$^{-1}$) was added to each well, and after 4 hours, supernatants were removed. Thereafter, 150 $\mu$L of DMSO was added to each well, and after sufficient shaking and complete color development, an absorbance value of each well was measured at a wavelength of 490 nm using a microplate reader. The relative cell viability was calculated according to the following formula:

Relative cell viability (%) = (average absorbance value at each concentration $\div$ average absorbance value of control group) $\times$ 100%.

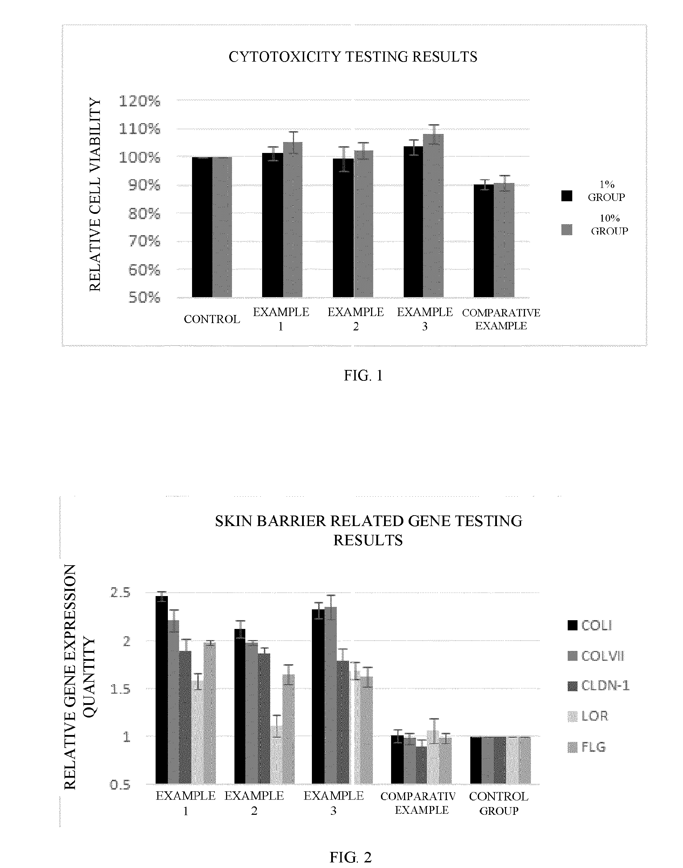

Experimental results are as follows. The cytotoxicity testing result of each example is shown in FIG. 1, and the relative cell viability in all the examples was equal to or larger than 80% within the test concentration range of 10% and 1% obtained by dilution, indicating that each example had no obvious cytotoxicity to HSF cells.

Example 5 Testing for skin barrier repairing function of the formulations

The integrity of the skin barrier function is the primary condition to ensure skin health, and the realization of skin barrier functions mainly depends on various proteins produced by healthy cells. The various proteins include various types of collagen, tight junction protein (CLDN-1), loricrin (LOR), filaggrin (FLG), or the like.
The experimental method is as follows. A real-time fluorescent quantitative PCR method is adopted. Human keratinocytes (HaCat cells) in the logarithmic growth phase were seeded into a 6-well plate at a seeding density of $6 \times 10^5$ cells/well, and incubated in an incubator (37°C, 5% $CO_2$) overnight. When the efficiency of plating cells in the 6-well plate reached 40% to 50%, the cell autophagy compositions were administered in groups in 2 mL per well to reach an action concentration of 1%, and each group corresponded to one well. After the administration was completed, the 6-well plate was placed in an incubator (37°C, 5% $CO_2$), and the cells were incubated and cultured for 24 hours. After the culture was completed, the cells were washed 3 times by adding phosphate-buffered saline (PBS) in 2 mL/well each time, and 1 mL of RNAiso Plus was added to each well. The cells were lysed by pipetting, and then cell lysates were collected and subjected to RNA extraction, reverse transcription, and fluorescent quantitative PCR to detect levels of Collagen Type I (COLI) genes and Collagen Type VII (COLVII) genes, and the results were calculated by $2^{-\Delta\Delta CT}$ method.

Experimental results are as follows. At the action concentration of 1%, compared with the control group, expression levels of COLI genes, COLVII genes, CLDN-1 genes, LOR genes, and FLG genes in each formulation show improvements in different degrees, indicating that the composition can promote the generation of collagen, tight junction proteins, loricrin, and filaggrin, and has a good effect on repairing and improving the skin barrier function. The results are shown in FIG. 2.

Example 6 Testing for enhancing skin cell autophagy function of the formulations

ATG5 is a key protein involved in the extension of a phagocytic membrane in autophagic vacuoles and forms a constitutive complex with ATG12. The ATG7 gene encodes an e1-like activating enzyme that is crucial for autophagy and cytosolic-to-vacuole trafficking. LC3, also known as microtubule-associated protein 1 light chain 3 (MAP1LC3), is a homolog of ATG8 in mammals. LC3 includes two interconvertible forms, LC3-I and LC3-II, which are involved in the formation of an autophagosome membrane. LC3-II is an important marker molecule of autophagosomes and increases with an increase of autophagosome membranes. LAMP2 is a component of the lysosomal membrane and is one of markers of cell autophagy, as a lysosomal marker. The changes in cell autophagy functions can be reflected by detecting the expression of the above and other genes in cells.

The experimental method is as follows. A real-time fluorescent quantitative PCR method is adopted. Human keratinocytes (HaCat cells) in the logarithmic growth phase were seeded into a 6-well plate at a seeding density of $6 \times 10^5$ cells/well, and incubated in an incubator (37°C, 5% $CO_2$) overnight. When the efficiency of plating cells in the 6-well plate reached 40% to 50%, the cell autophagy compositions were administered in groups in 2 mL per well to reach an action concentration of 1%, and each group corresponded to one well. After the administration was completed, the 6-well plate was placed in an incubator (37°C, 5% $CO_2$), and the cells were incubated and cultured for 24 hours. After the culture was completed, the cells were washed 3 times by adding PBS in 2 mL/well each time, and 1 mL of RNAiso Plus was added to each well. The cells were lysed by pipetting, and then cell lysates were collected and subjected to RNA extraction, reverse transcription, and fluorescent quantitative PCR to detect levels of ATG5 genes, ATG7 genes, LC3-II genes, and LAMP2 genes, and the results were calculated by $2^{-\Delta\Delta CT}$ method.

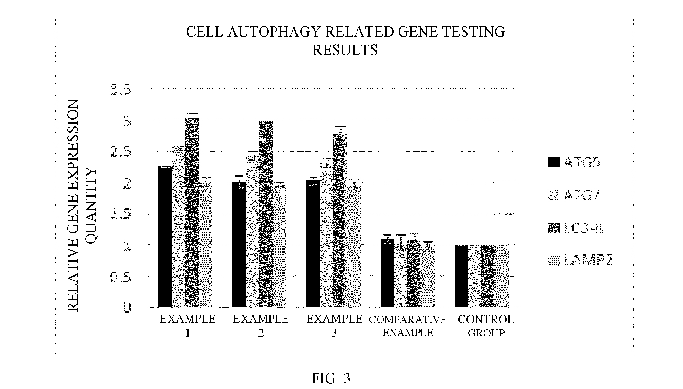

Experimental results are as follows. At the action concentration of 1%, compared with the control group, expression levels of ATG5 genes, ATG7 genes, LC3-II genes, and LAMP2 genes in each formulation show improvements in different degrees, indicating that the composition can significantly improve skin cell autophagy. The results are shown in FIG. 3.

Example 7 Human patch test for anti-aging function of the formulations

Reference method: "Safety and Technical Standards for Cosmetics (Version 2015)".

A qualified patch test device with an area not exceeding 50 mm² and a depth of about 1 mm was selected. Each of four test samples was placed into a small chamber of the patch test device in an amount of about 0.02 mL to 0.025 mL (liquid). The patch test devices with the test samples were applied to the curved sides of the forearms of subjects (in which 30 subjects correspond to each kind of test sample) with hypoallergenic tapes, and the patch test devices were evenly applied to the skin by gentle pressing and kept on the skin for 24 hours. The cutaneous reaction was observed 30 minutes (after the indentation disappeared), 24 hours and 48 hours after removing the patch test devices with the test samples, and observation results were recorded. The results are shown in Table 2.

Table 2 Human patch test results

| Sequence number | Sample name | Human skin patch results |
| --- | --- | --- |
| 1 | Example 1 | 0 case of adverse reactions |
| 2 | Example 2 | 0 case of adverse reactions |
| 3 | Example 3 | 0 case of adverse reactions |
| 4 | Comparative Example | 0 case of adverse reactions |

The patch test results show that Examples 1 to 3 and Comparative Example prepared by the present disclosure are safe and do not cause adverse reactions in the human body.

**Claims**

1. A cell autophagy composition, having a composition of, by mass percent:

   0.05% to 0.5% of collagen;
   0.5% to 3% of a yeast fermentation product extract;
   0.01% to 0.5% of acetyl hexapeptide-8;
   0.01% to 0.5% of palmitoyl hexapeptide-12;
   0.01% to 0.5% of dipeptide-15;
   0.1% to 2% of tripeptide-1;
   0.01% to 0.5% of dipeptide-1;
   5% to10% of 1,2-hexanediol; and
   0.05% to 0.5% of ethylhexylglycerin,
   with the remainder being water;
   wherein the yeast fermentation product extract is Pichia pastoris fermentation product extract.

2. The cell autophagy composition according to claim 1, wherein the collagen is one or both of type I collagen and type III collagen.

3. A preparation method for the cell autophagy composition according to any one of claims 1 to 2, the method comprising:

   step 1 of dissolving the collagen and the yeast fermentation product extract in water and stirring evenly to obtain a solution;
   step 2 of adding acetyl hexapeptide-8, palmitoyl hexapeptide-12, dipeptide-15, tripeptide-1, and dipeptide-1 into the solution obtained in step 1 and stirring evenly to obtain a solution; and
   step 3 of filtering and sterilizing the solution obtained in step 2 to obtain the cell autophagy composition.

4. An application of the cell autophagy composition according to any one of claims 1 to 2 in preparation of cosmetics.

5. The application of the cell autophagy composition in preparation of cosmetics according to claim 4, wherein the cell autophagy composition is mixed with a cosmetic matrix to obtain a cosmetic with cell autophagy function, the cosmetic matrix is any one of an aqueous solution, a cream, a facial mask, and an essence, and an addition amount of the cell autophagy composition is 1% to10% of the mass percentage of the cosmetic.

**Patentansprüche**

1. Zellautophagie-Zusammensetzung mit einer Zusammensetzung in Gewichtsprozent von

   0,0.5 % bis 0,5 % Kollagen,
   0,5 % bis 3 % eines Hefefermentationsproduktextrakts;
   0,01 % bis 0,5 % Acetylhexapeptid-8;
   0,01 % bis 0,5 % Palmitoylhexapeptid-12;
   0,01 % bis 0,5 % Dipeptid-15;
   0,1 % bis 2 % Tripeptid-1;
   0,01 % bis 0,5 % Dipeptid-1;
   5 % bis 10 % 1,2-Hexandiol und
   0,05 % bis 0,5 % Ethylhexylglycerin,
   wobei der Rest Wasser ist,
   wobei der Hefefermentationsproduktextrakt ein Hefefermentationsproduktextrakt von Pichia pastoris ist.

2. Zellautophagie-Zusammensetzung nach Anspruch 1, wobei das Kollegen ein Kollagen vom Typ I und/oder vom Typ III ist.

3. Herstellungsverfahren für die Zellautophagie-Zusammensetzung nach einem der Ansprüche 1 bis 2, wobei das Verfahren

   als Schritt 1 das Auflösen des Kollagens und des Hefefermentationsproduktextrakts in Wasser und gleichmäßiges Rühren zum Erhalten einer Lösung,
als Schritt 2 das Hinzufügen von Acetylhexapeptid-8, Palmitoylhexapeptid-12, Dipeptid-15, Tripeptid-1 und Dipeptid-1 zu der im Schritt 1 erhaltenen Lösung und gleichmäßiges Rühren zum Erhalten einer Lösung und als Schritt 3 das Filtrieren und Sterilisieren der im Schritt 2 erhaltenen Lösung umfasst, um die Zellautophagie-Zusammensetzung zu erhalten.

4. Verwendung der Zellautophagie-Zusammensetzung nach einem der Ansprüche 1 bis 2 in der Herstellung von Kosmetika.

5. Verwendung der Zellautophagie-Zusammensetzung in der Herstellung von Kosmetika nach Anspruch 4, wobei die Zellautophagie-Zusammensetzung mit einer Kosmetikmatrix gemischt wird, um ein Kosmetikum mit Zellautophagie-Funktion zu erhalten, wobei die Kosmetikmatrix eine wässrige Lösung, eine Creme, eine Gesichtsmaske oder eine Essenz ist und die zugegebene Menge der Zellautophagie-Zusammensetzung 1 % bis 10 % des Massenanteils des Kosmetikums beträgt.

**Revendications**

1. Composition d'autophagie cellulaire, comprenant, en pourcentage de masse :

0,05 % à 0,5 % de collagène ;
0,5 % à 3 % d'un extrait de produit de fermentation de levure ;
0,01 % à 0,5 % d'acétyl-hexapeptide-8 ;
0,01 % à 0,5 % de palmitoyl-hexapeptide-12 ;
0,01 % à 0,5 % de dipeptide-15 ;
0,1 % à 2 % de tripeptide-1 ;
0,01 % à 0,5 % de dipeptide-1 ;
5 % à 10 % de 1,2-hexanediol ; et
0,05 % à 0,5 % d'éthylhexylglycérine,
le reste étant de l'eau ; dans lequel l'extrait de produit de fermentation de levure est un extrait de produit de fermentation de Pichia pastoris.

2. Composition d'autophagie cellulaire selon la revendication 1, dans laquelle le collagène est du collagène de type I, du collagène de type III ou les deux.

3. Procédé de préparation de la composition d'autophagie cellulaire selon l'une quelconque des revendications 1 à 2, ledit procédé comprenant :

l'étape 1 consistant à dissoudre le collagène et l'extrait de produit de fermentation de levure dans de l'eau et à agiter uniformément pour obtenir une solution ;
l'étape 2 consistant à ajouter de l'acétyl-hexapeptide-8, du palmitoyl-hexapeptide-12, du dipeptide-15, du tripeptide-1 et du dipeptide-1 dans la solution obtenue à l'étape 1 et à agiter uniformément pour obtenir une solution ; et l'étape 3 consistant à filtrer et à stériliser la solution obtenue à l'étape 2 pour obtenir la composition pour l'autophagie cellulaire.

4. Utilisation de la composition d'autophagie cellulaire selon l'une quelconque des revendications 1 à 2 dans la fabrication de produits cosmétiques.

5. Utilisation de la composition d''autophagie cellulaire dans la préparation de produits cosmétiques selon la revendication 4, dans laquelle la composition d'autophagie cellulaire est mélangée à une matrice cosmétique afin d'obtenir un produit cosmétique doté d'une fonction favorisant l'autophagie cellulaire, la matrice cosmétique étant choisie parmi une solution aqueuse, une crème, un masque facial et une essence, et la quantité ajoutée de la composition d'autophagie cellulaire représentant 1 % à 10 % du pourcentage de masse du produit cosmétique.

FIG. 1

FIG. 2

CELL AUTOPHAGY RELATED GENE TESTING RESULTS

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 115851466